(19) Europäisches Patentamt
European Patent Office
Office européen des brevets

(11) Numéro de publication : **0 433 124 A1**

(12) # DEMANDE DE BREVET EUROPEEN

(21) Numéro de dépôt : **90403378.4**

(51) Int. Cl.$^5$ : **C07C 255/50,** C07C 63/70

(22) Date de dépôt : **28.11.90**

(30) Priorité : **11.12.89 FR 8916351**

(43) Date de publication de la demande :
**19.06.91 Bulletin 91/25**

(84) Etats contractants désignés :
**AT BE CH DE DK ES GB GR IT LI LU NL SE**

(71) Demandeur : **ISOCHEM (société anonyme)**
**10, rue Clément Marot**
**F-75008 Paris (FR)**

(72) Inventeur : **Wirth, Didier**
**3, Place du Palais Bourbon**
**F-75007 Paris (FR)**
Inventeur : **Gibert, Dominique**
**12, rue de l'Eglise**
**F-60340 Villers-S/Saint-Leu (FR)**
Inventeur : **Boutin, Annie**
**49, Boulevard du Général Leclerc**
**F-92110 Clichy (FR)**

(74) Mandataire : **Battut, Michel**
**OFFICE PICARD 134, boulevard de Clichy**
**F-75018 Paris (FR)**

(54) **Dichloro-2,4-fluoro-5-benzonitrile et procédés pour sa préparation, son application à la préparation de l'acide chloro-2-difluoro-4,5-benzoïque et nouveau procédé de préparation dudit acide.**

(57) L'invention concerne le dichloro-2,4-fluoro-5-benzonitrile, de formule :

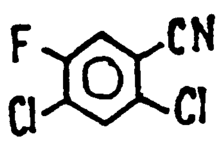

que l'on utilise comme produit de base pour la synthèse de l'acide chloro-2-difluoro-4,5-benzoïque, par passage au chloro-2-difluoro-4,5-benzonitrile de formule :

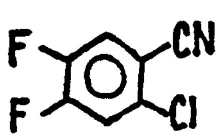

obtenu par fluoruration sélective.

Application à la préparation de l'acide chloro-2-difluoro-4,5-benzoïque par hydrolyse du chloro-2-difluoro-4,5-benzonitrile en milieu aqueux acide.

EP 0 433 124 A1

# DICHLORO-2,4-FLUORO-5-BENZONITRILE ET PROCEDES POUR SA PREPARATION, SON APPLICATION A LA PREPARATION DE L'ACIDE CHLORO-2-DIFLUORO-4,5-BENZOIQUE ET NOUVEAU PROCEDE DE PREPARATION DUDIT ACIDE

La présente invention se rapporte au dichloro-2,4-fluoro-5-benzonitrile, à des procédés pour sa préparation, à son application à la préparation de l'acide chloro-2-difluoro-4,5-benzoïque selon un nouveau procédé.

La présente invention a donc aussi pour objet des procédés d'obtention des intermédiaires nouveaux rencontrés lors de la mise en oeuvre d'un nouveau procédé de préparation de l'acide chloro-2-difluoro-4,5-benzoïque. Ces produits constituent des matières premières utiles dans les domaines pharmaceutique et agrochimique.

L'invention a en particulier pour objet la préparation du dichloro-2,4-fluoro-5-benzonitrile, corps nouveau. Elle a aussi pour objet la préparation du chloro-2-difluoro-4,5-benzonitrile, corps nouveau, obtenu à partir du précédent et utilisé comme intermédiaire dans la préparation de l'acide chloro-2-difluoro-4,5-benzoïque.

On sait que des acides quinolone-3-carboxyliques constituent une classe importante d'agents antibactériens de formule générale (I) :

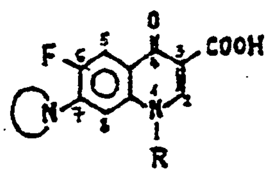

caractérisée par la présence d'un atome de fluor en position 6 sur le noyau quinoléine, par celle d'un substituant aminé cyclique ou hétérocyclique en position 7 ainsi que par la substitution au niveau de l'atome d'azote en position 1 au moyen d'un radical alcoyle, cycloalcoyle ou aromatique.

On obtient classiquement les composés (I) en plusieurs étapes à partir d'un acide benzoïque de formule (II) :

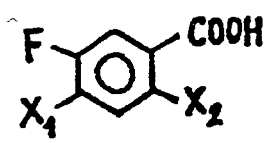

dans laquelle $X_1$, désigne un atome de fluor ou de chlore et $X_2$ désigne un atome de fluor, de chlore ou de brome. Pour des raisons de réactivité, on préfère utiliser des intermédiaires du type (II) dans la formule desquels $X_1$ est un atome de fluor et plus particulièrement l'acide chloro-2-difluoro-4,5-benzoïque (III)

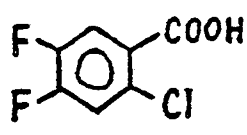

La littérature chimique mentionne plusieurs voies de synthèse pour ce composé et notamment les procédés décrits dans les documents suivants :

a) Le brevet japonais n° 108839/1987, decrit l'hydrolyse d'un précurseur Trifluorométhylé de cet acide, suivant la réaction :

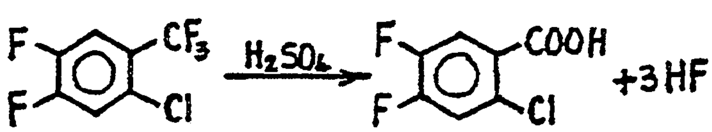


b) Le brevet des Etats-Unis d'Amérique n° 4833270, présente une synthèse en quatre étapes, à partir

de la 3,4-difluoroaniline ;

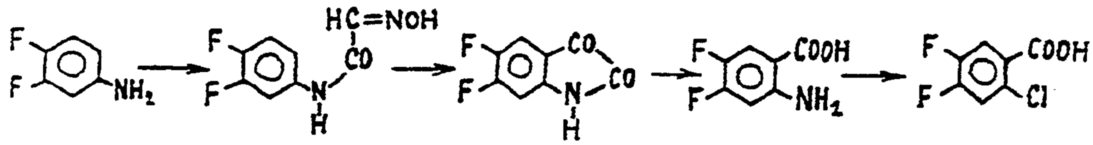

c) Le brevet européen n° 303291 décrit une synthèse en deux étapes :

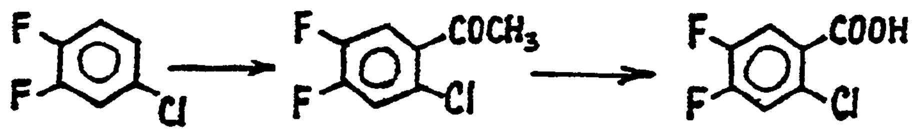

Du point de vue, industriel ces trois voies apparaissent peu satisfaisantes car elles font appel à des matières premières très coûteuses dans leur ensemble. Par ailleurs, le dégagement de fluorure d'hydrogène observé dans le premier cas interdit l'utilisation de réacteurs conventionnels.

L'objet de la présente invention est donc de permettre l'obtention de l'acide chloro-2-difluoro-4,5-benzoïque (III) dans des conditions économiques et technologiques plus acceptables. Ainsi, on opère en deux étapes seulement, partant du dichloro-2,4-fluoro-5-benzonitrile (IV) corps nouveau et très accessible.

Dans un premier procédé de préparation du composé nouveau de formule (IV) on met en oeuvre les étapes chimiques suivantes :

a) bromation du dichloro-2,4-fluorobenzène, pour obtenir le bromo-5-dichloro-2,4 fluorobenzène

b) action d'un cyanure sur le bromo-5-dichloro-2,4 fluorobenzène, pour obtenir le dichloro-2,4-fluoro-5-benzonitrile.

Dans un second procédé de préparation du composé de formule (IV), on met en oeuvre les étapes chimiques suivantes :

a) On part de l'acide dichloro-2,4-fluoro-5-benzoïque, que l'on traite par le chlorure de thionyle, pour obtenir le chlorure d'acide correspondant ;

b) On traite le chlorure d'acide par l'ammoniaque, pour obtenir l'amide correspondant ;

c) On traite l'amide par l'oxychlorure de phosphore, pour obtenir le dichloro-2,4-fluoro-5-benzonitrile.

Le nouveau procédé de préparation de l'acide chloro-2-difluoro-4,5-benzoïque, selon la présente invention, met en oeuvre les étapes chimiques suivantes :

a) passage du dichloro-2,4-fluoro-5-benzonitrile au chloro-2-difluoro-4,5-benzonitrile par échange sélectif d'halogène, sous l'action d'au moins un fluorure alcalin,

b) hydrolyse du chloro-2-difluoro-4,5-benzonitrile en acide chloro-2-difluoro-4,5 benzoïque en milieu aqueux acide.

Le procédé de préparation de l'acide chloro-2-difluoro-4,5-benzoïque (V) ci-dessus résumé, est défini plus en détail dans la description ci-après :

a) dans un premier temps on convertit le dichloro-2,4-fluoro-5-benzonitirile (IV) en chloro-2-difluoro-4,5-benzonitrile (V) par action d'ion fluorure :

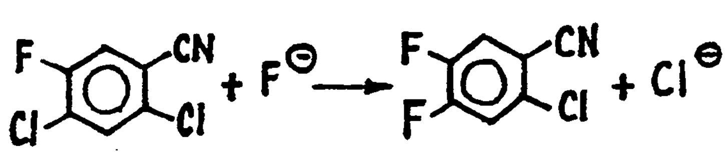

(IV) (V)

On observe à ce propos une sélectivité inattendue et le benzonitrile (V) obtenu n'est accompagné que de faibles quantités de son isomère, le chloro-4-difluoro-2,5-benzonitrile et de trifluoro-2,4,5-benzonitrile, produit de fluoruration complète.

La réaction d'échange a lieu en présence d'un fluorure alcalin tel que les fluorures de sodium, potassium, rubidium ou césium, seuls ou en mélange et de préférence dans un solvant polaire aprotique tel que le N,N-diméthyl formamide (DMF), la N-méthyl pyrrolidone, le diméthyl sulfoxyde, la diméthyl sulfone ou le tétraméthylène sulfone, éventuellement additionnés d'un autre solvant moins polaire tel que l'heptane, le toluène, le xylène ou le chlorobenzène. La présence d'un catalyseur, sans être préjudiciable, n'est pas nécessaire ici car la réaction d'échange est assez facile aussi pour que des températures comprises entre 100° et 180°C suffisent pour la mener à son terme. Enfin, la présence d'eau en trop grande quantité est évitée.

Le chloro-2-difluoro-4,5-benzonitrile ainsi obtenu peut être purifié par distillation et recristallisation dans un hydrocarbure par exemple, sans que ceci représente une nécessité du procédé.

b) dans un deuxième temps l'hydrolyse du chloro-2-difluoro-4,5-benzonitrile ((V) conduit à l'acide chloro-2-difuloro-4,5-benzoïque (III)

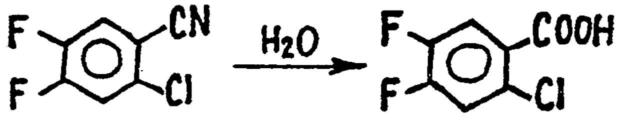


On opère à une température comprise entre 100°C et 200°C, sous pression ambiante ou supèrieure selon les cas. On préfère une catalyse acide et conviennent ici les acides chlorhydrique, bromhydrique, sulfurique, phosphorique ou méthanesulfonique par exemple. L'acide recherché est ensuite purifié par les moyens classiques tels le passage à un sel, la décoloration à l'aide de charbon actif d'une solution ou la recristallisation.

En ce qui concerne le dichloro-2,4-fluoro-5-benzonitrile (IV) utilisé comme matière première, il est aisément accessible à partir d'intermédiaires commerciaux.

La bromation du dichloro-2,4-fluorobenzène, suivie de l'action du cyanure cuivreux, constitue une voie d'accès particulièrement appréciée.

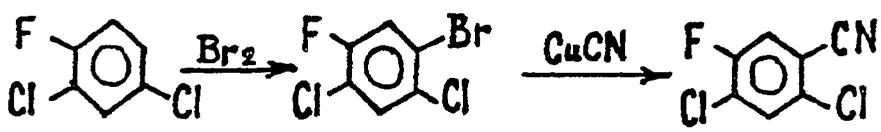


D'autres voies telles que le passage de l'acide dichloro-2,4-fluoro-5-benzoïque au nitrile correspondent, par exemple via le chlorure d'acide, puis l'amide, conviennent aussi.

Les quelques exemples suivants illustrent davantage l'invention, sans pour autant en limiter la portée.

Exemple 1

Préparation du dichloro-2,4-fluoro-5-benzonitrile :

a) dans un réacteur de 10 l, on porte au reflux pendant 5 heures un mélange de 2,5 kg d'acide dichloro-2,4-fluoro-5-benzoïque, 3 l de chlorure de thionyle et 0,05 l de diméthylformamide (DMF). On concentre ensuite d'abord sous pression ambiante, puis sous une pression de 50 mm Hg, jusqu'à atteindre une température de masse de 70°C.

b) On introduit le résidu en 2 heures dans un réacteur de 20 l, contenant 10 l d'ammoniaque à 20% refroidis vers 0°C. On agite pendant 12 heures en laissant la température remonter vers 20°C. On essore et rince abondamment à l'eau, puis sèche pendant 15 heures le produit à l'air.

c) On charge l'amide et 6 l de toluène dans un réacteur de 20 l puis porte au reflux. Quand la température de la masse atteint 113°C, on coule 560 ml d'oxychlorure de phosphore. On maintient pendant 6 heures encore au reflux, puis refroidit vers +5°C. On reprend à l'eau, neutralise par addition de lessive de soude, puis concentre la solution toluénique sous pression réduite jusqu'à atteindre une température de masse de 80°C.

Le résidu est cristallisé dans l'heptane et le solide séché à l'air pendant 15 heures : on obtient 1,886 kg de dichloro-2,4-fluoro-5-benzonitrile, soit un rendement de 82,7%.

- point de fusion 48°C
- point d'ébullition 120°C sous 16 mm Hg

Exemple 2

Préparation du dichloro-2,4-fluoro-5-benzonitrile :

a) Dans un réacteur thermostatique de 5 l on charge 660 g de dichloro-2,4 fluorobenzène et 2,24 g de

fer en poudre, puis porte à 30°C.

On coule 704 g de brome, puis porte à 50°C et reprend la masse dans 660 ml de chloroforme.

On lave à l'eau, puis avec une solution de bisulfite de sodium et finalement avec une solution de carbonate de sodium.

On concentre sous vide et cristallise le résidu dans l'heptane ce qui permet d'obtenir en quatre jets 683 g de bromo-5-dichloro-2,4,-fluorobenzène, soit un rendement de 70%

- point de fusion 66,5°C
- point d'ébullition 110°C sous 18 mm Hg

b) On porte pendant 8 heures à 120°C un mélange de 244 g du composé bromé précédent et 134,25 g de cyanure cuivreux en suspension dans 600 ml de N méthyl pyrrolidone. On distille ensuite sous le vide de la trompe à eau, en présence d'huile de paraffine. L'analyse du distillat permet d'évaluer le rendement en dichloro-2,4-fluoro-5-benzonitrile aux environs de 78,3% et la quantité de dérivé bromé restante à 3% de la quantité initiale.

Une distillation fine conduit au nitrile recherché pur

- Point d'ébullition 120°C sous 16 mm Hg
- Point de fusion 48°C

Exemple 3

Préparation du chloro-2-difluoro-4,5-benzonitrile :

Dans un réacteur de 500 ml on charge 47,5g de dichloro-2,4-fluoro-5-benzonitrile, 14,5 g de fluorure de potassium, 1,9 g de fluorure de césium, 190 ml de tétraméthylène sulfone et 100 ml de toluène.

On porte au reflux et distille assez de toluène pour que la température de masse atteigne 150°C, puis maintient à cette température pendant 15 heures.

On refroidit vers 50°C, puis concentre sous pression de 20 mm Hg environ, jusqu'à observer une température de masse de 150°C.

L'analyse du distillat permet de conclure :

- rendement en trifluoro-2,4,5-benzonitrile 10%
- rendement en chloro-2-difluoro-4,5-benzonitrile 52%
- rendement en chloro-4-difluoro-2,5 benzonitrile 3,3%

Une redistillation fine permet d'isoler le chloro-2-difluoro-4,5-benzonitrile, purifiable par recristallisation dans l'heptane.

- point d'ébullition 95°C sous 16 mm Hg
- point de fusion 36,2°C

Exemple 4

Préparation du Chloro-2-difluoro-4,5-benzonitrile

On procède suivant le même mode opératoire que dans l'exemple 3, en utilisant cette fois une quantité de fluorure de potassium supérieure de 50% et l'on opère pendant 6 heures à 180°C. On observe alors les rendements suivants :

- Trifluoro-2,4,5-benzonitrile 20%
- chloro-2-difluoro-4,5-benzonitrile 70%
- chloro-4-difluoro-2,5-benzonitile 2%

Exemple 5

Préparation du chloro-2-difluoro-4,5-benzonitrile

On procède suivant le même mode opératoire que dans l'exemple 3, en utilisant cette fois une quantité double de fluorure de potassium de celle indiquée dans l'exemple 4, mais sans fluorure de césium. On opère pendant 15 heures à 180°C. On peut alors observer les rendements suivants :

- dichloro-2,4-fluoro-5-benzonitrile inchangé 12%
- Trifluoro-2,4,5-benzonitrile 12%
- 2 chlorodifluorobenzonitriles isomères 61% au total

Exemple 6

Préparation du chloro-2-difluoro-4,5-benzonitrile

On opère suivant le même mode opératoire dans l'exemple 3, en remplaçant cette fois la tétraméthylène sulfone par le DMF. On peut ainsi obtenir le chloro-2-difluoro-4,5-benzonitrile avec un rendement de 81% par rapport au produit de départ consommé, au bout de 9 heures.

Exemple 7

Préparation de l'acide chloro-2-difluoro-4,5-benzoïque

On porte pendant 150 mn à 135°C un mélange de 80 g de chloro-2-difluoro-4,5-benzonitrile et 200 ml d'acide sulfurique à 75%. On refroidit, dilue à l'eau et extrait au chlorure de méthylène. La solution chlorométhylénique est ensuite concentrée en présence d'eau : l'acide recherché cristallise. On essore et rince à l'eau, puis sèche pendant 15 heures sous vide à 50°C.

On obtient ainsi 68 g d'acide chloro-2-difluoro-4,5-benzoïque de bonne qualité, soit un rendement de 94%.

• point de fusion 107°C.

## Revendications

1. Dichloro-2,4-fluoro-5-benzonitrile, de formule :

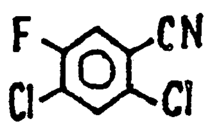

2. Procédé de préparation du composé nouveau selon la revendication 1, caractérisé par la mise en oeuvre des étapes chimiques suivantes :
   a) bromation du dichloro-2,4-fluobenzène, pour obtenir le bromo-5-dichloro-2,4 fluorobenzène ;
   b) action d'un cyanure sur le bromo-5-dichloro-2,4 fluorobenzène, pour obtenir le dichloro-2,4-fluoro-5-benzonitrile.

3. Procédé de préparation du composé nouveau selon la revendication 1, caractérisé par la mise en oeuvre des étapes chimiques suivantes :
   a) On part de l'acide dichloro-2,4-fluoro-5-benzoïque, que l'on traite par le chlorure de thionyle, pour obtenir le chlorure d'acide correspondant ;
   b) On traite le chlorure d'acide par l'ammoniaque, pour obtenir l'amide correspondant ;
   c) On traite l'amide par l'oxychlorure de phosphore, pour obtenir le dichloro-2,4-fluoro-5-benzonitrile.

4. Application du dichloro-2,4-fluoro-5-benzonitrile selon la revendication 1, en tant que produit de base nouveau dans la préparation de l'acide chloro-2-difluoro-4,5-benzoïque.

5. Application du dichloro-2,4-fluoro-5-benzonitrile selon la revendication 1 à la préparation du chloro-2-difluoro-4,5-benzonitrile, en tant qu'intermédiaire dans la préparation de l'acide chloro-2-difluoro-4,5-benzoïque.

6. Chloro-2-difluoro-4,5-benzonitrile, de formule :

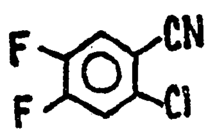

7. Procédé de préparation du chloro-2-difluoro-4,5-benzonitrile selon la revendication 6, caractérisé par la conversion du dichloro-2,4-fluoro-5 benzonitrile par action sélective d'un ion fluorure à partir d'au moins un fluorure alcalin choisi parmi les fluorures de sodium, potassium, rubidium et/ou cesium, dans un solvant polaire aprotique, éventuellement additionné d'un autre solvant, en présence d'un catalyseur facultativement.

8. Procédé de préparation de l'acide chloro-2-difluoro-4,5 benzoïque, caractérisé par la mise en oeuvre des étapes chimiques suivantes, prises ensemble ou séparément :
   a) passage du dichloro-2,4-fluoro-5-benzonitrile au chloro-2-difluoro-4,5-benzonitrile par échange sélectif d'halogène, sous l'action d'au moins un fluorure alcalin,
   b) hydrolyse du chloro-2-difluoro-4,5-benzonitrile en acide chloro-2-difluoro-4,5 benzoïque en milieu aqueux acide.

Office européen des brevets

RAPPORT DE RECHERCHE EUROPEENNE

Numero de la demande

EP 90 40 3378

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 303 291 (ASAHI GLASS)<br>* Pages 4,5 *<br>--- | 1-8 | C 07 C 255/50<br>C 07 C 63/70 |
| D,A | US-A-4 833 270 (P. BITHA)<br>* Colonnes 1,2 *<br>----- | 1-8 | |

| DOMAINES TECHNIQUES RECHERCHES (Int. Cl.5) |
|---|
| C 07 C 255/00 |

Le présent rapport a été établi pour toutes les revendications

| Lieu de la recherche | Date d'achèvement de la recherche | Examinateur |
|---|---|---|
| LA HAYE | 18-03-1991 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)